# EUROPEAN PATENT SPECIFICATION

(11) **EP 3 478 304 B1**
(45) Date of publication and mention of the grant of the patent: **23.02.2022**
(21) Application number: 16907546.2
(22) Date of filing: 30.06.2016
(51) Int. Cl.: A61K 35/742, C12N 1/20, A61P 1/00, A61P 25/24

(54) **BACILLUS COAGULANS MTCC 5856 FOR THE MANAGEMENT OF MAJOR DEPRESSIVE DISORDER**
BACILLUS COAGULANS MTCC 5856 ZUR BEHANDLUNG VON SCHWEREN DEPRESSIONEN
BACILLUS COAGULANT MTCC 5856 POUR LA PRISE EN CHARGE DU TROUBLE DÉPRESSIF MAJEUR

(43) Date of publication of application: 08.05.2019
(73) Proprietor: Sami Labs Limited, Bangalore 560058 KRN (IN)
(72) Inventor: MAJEED, Muhammed, East Windsor, NJ 08520 (US); NAGABHUSHANAM, Kalyanam, East Windsor, NJ 08520 (US); ARUMUGAM, Sivakumar, Bangalore 560058 (IN); ALI, Furqan, Bangalore 560058 (IN)
(74) Representative: Dr. Gassner & Partner mbB
(86) International application number: PCT/US2016/040290
(87) International publication number: WO 2018/004586

(56) References cited:
- US-A1- 2013 022 577
- US-A1- 2015 173 374
- US-A1- 2015 202 238
- US-A1- 2016 129 054
- MUHAMMED MAJEED ET AL: "Bacillus coagulans MTCC 5856 supplementation in the management of diarrhea predominant Irritable Bowel Syndrome: a double blind randomized placebo controlled pilot clinical study", NUTRITION JOURNAL, vol. 15, no. 1, 27 February 2016 (2016-02-27), pages 1-10, XP055396406, DOI: 10.1186/s12937-016-0140-6
- Madhu G. Soni: "GRAS Notification for Bacillus coagulans spore preparation (LactoSpore )", , 15 September 2015 (2015-09-15), XP055644425, Retrieved from the Internet: URL:https://www.fda.gov/media/95330/downlo ad [retrieved on 2019-11-20]
- HAN KYUNGSUN ET AL: "Efficacy of double-coated probiotics for irritable bowel syndrome: a randomized double-blind controlled trial", JOURNAL OF GASTROENTEROLOGY, SPRINGER JAPAN KK, JP, vol. 52, no. 4, 13 May 2016 (2016-05-13), pages 432-443, XP036191707, ISSN: 0944-1174, DOI: 10.1007/S00535-016-1224-Y [retrieved on 2016-05-13]
- MUHAMMED MAJEED ET AL: "Bacillus coagulans MTCC 5856 for the management of major depression with irritable bowel syndrome: a randomised, double-blind, placebo controlled, multi-centre, pilot clinical study", FOOD & NUTRITION RESEARCH, vol. 62, no. 0, 4 July 2018 (2018-07-04), XP055644428, DOI: 10.29219/fnr.v62.1218
- KUMAR, V ET AL.: 'To assess the effectiveness of study m edication LactoSporein' XP055451228 Retrieved from the Internet: <URL:http://ctri.nic.in/Clinicaltrials/show allp.php?mid1=11417&EncHid=&userName=CTRI/ 2015/05/005754>
- FITZPATRICK, LR ET AL.: 'Bacillus coagulans GBI-30, 6086 limits the recurrence of Clostridium difficile-Induced L;ulitis following vancomycin withdrawal in mice' GUT PATHOGENS vol. 4, no. 1, 22 October 2012, pages 1 - 9, XP021108243

## Description

### BACKGROUND OF THE INVENTION

**Field of the invention:** The invention in general relates to probiotics and their therapeutic potential. More specifically, the present invention relates to *Bacillus coagulans* MTCC 5856 for the treatment of major depressive disorder associated with irritable bowel syndrome.

**Description of prior art:** Major depressive disorder (MDD) is characterized by increased medical morbidity, mortality, functional impairment, reduced quality of life, substantial health-care costs, and an increased risk of suicide, loss of interest or pleasure, disturbed sleep or appetite, low energy, and feelings of guilt or low self-worth (Greden et al, J Clin Psychiat. 2001, 62:26-31; Tranter et al, J Psychiat Neurosci. 2002, 27:241-247; Uher et al, Depress Anxiety. 2014, 31:459-471). MDD is one of the most common mental disorders worldwide, with a life time prevalence of 16.2% and a 12-month prevalence of 6.6% in developed countries (Trivedi et al, CNS Spectr. 2007, 12:1-27). According to the World Health Organization (WHO, 2010), MDD carries the heaviest burden of disability among mental and behavioural disorders. The findings from the 2014 National Survey on Drug Use and Health (NSDUH) revealed that in 2014, 11.4 percent of youths aged 12 to 17 (2.8 million adolescents) had a major depressive episode (MDE) in the past year (Center for Behavioral Health Statistics and Quality, 2015). Youths aged 12 to 17 in 2014 who had a past year MDE were more likely than those without a past year MDE to have used any illicit drugs in the past year (33.0 vs. 15.2 percent). Nierenberg and DeCecco concluded that though the initial antidepressant therapy reduces symptoms of depression in patients, but only 50-60% of patients with major depressive disorder respond to therapy (Nierenberg and DeCecco, J Clin Psychiat. 2001, 62:5-9). Furthermore, between 30 and 40% of patients who suffer from MDD never achieve symptom resolution with standard antidepressant treatment (Amsterdam and HomigRohan, Psychiatr Clin North Am. 1996, 19:371-386.; Nierenberg and Amsterdam, J Clin Psychiat. 1990, 51:39-47). The association between MDD and altered gut microbiota is positioned within the concept of brain-gut microbe bidirectional signalling and its role in body homeostasis. A stable gut microbiota is cited to be essential for normal gut physiology and is believed to contribute to appropriate signalling along the brain-gut axis leading to the healthy status of an individual. Conversely intestinal dysbiosis can adversely influence gut physiology leading to inappropriate gut-brain signalling and associated deleterious consequences for normal CNS functions in individuals. (Sue Grenham et al, Front Physiol. 2011, 2:1-15). While in general it may be understood that "probiotics" are organisms or their substances that contribute to intestinal microbial balance thus conferring a health benefit to the host (FAO / WHO, 2002 in combination with Fuller R, Gut 1991, 32 (4): 439-442), and that the modification of microbial ecology by probiotic therapy may be used therapeutically for the management of stress responses and symptoms of anxiety and depression (Krabbe et al, Brain Behav Immun. 2005, 19:453-460; Ait-Belgnaoui et al, Psychoneuroendocrinology. 2012, 37:1885-95; De Palma et al, J Physiol. 2014, 592: 2989-2997), to produce an effective probiotic it is necessary to establish specificity of the microbes responsible for this effect in a fully conventional animal (Fuller R, Gut 1991, 32 (4): 439-442). Rather, a specific probiotic microbe, its ability to function as a protective gut microflora and further, its ability to confer a specific associated health benefit to an animal must be fully characterised and studied. Vasiljevic et al, Int. Dairy J. 2008, 18:714-728 indicated that the health benefits of probiotics are very strain specific and there is no single universal strain that would provide all proposed benefits and not all strains of the same species can effectively be used for treating against a defined health condition. The individual strains have to be tested for each property since probiotic properties are generally strain specific (Verdenelli et al, Eur J Nutr. 2009, 48(6):355-63). Probiotic effect for the production of a bioactive for specific immunomodulatory or neurological effects (Hyland & Stanton. Editor(s): The Gut-Brain Axis, (2016) 1st Edition: Dietary, Probiotic, and Prebiotic Interventions on the Microbiota), weight regulation (Million et al, Microb Pathog. 2013; 55:52-54.) are reported to be species and strain specific. Lack of complete evidence for each potential probiotic strain in terms of its protective effect and specific health benefit thereof is a technical problem in the field of probiotics therapy.

The present invention solves one such technical problem in establishing in vivo evidence for the protective effect of *Bacillus coagulans* MTCC 5856 in Irritable Bowel Syndrome (IBS) associated Major Depressive Disorder (MDD). It is thus the principle objective of the present invention to disclose the efficacy of *Bacillus coagulans* MTCC 5856 in ameliorating the symptoms of Major Depressive Disorder (MDD) in patients with Irritable Bowel Syndrome (IBS). While in the instant case, MDD associated with IBS is presented as an illustrative example, the invention also encompasses the therapeutic effect of *Bacillus coagulans* MTCC 5856 in the management of MDD in general.

The present invention fulfils the aforesaid objective and provides further related advantages.

### SUMMARY OF THE INVENTION

The present invention is defined in the claims and is directed to Bacillus coagulans MTCC 5856 for use in a method of therapeutically managing major depressive disorder associated with Irritable Bowel Syndrome in a mammal, said method consists of step of administering an effective dose of Bacillus coagulans MTCC 5856 to said mammal experiencing a symptom of major depressive disorder, wherein the symptom of major depressive disorder is one selected from the group consisting of sleep disorder and dementia.

Other features and advantages of the present invention will become apparent from the following more detailed description, taken in conjunction with the accompanying images, which illustrate, by way of example, the principle of the invention.

### BRIEF DESCRIPTION OF THE DRAWINGS

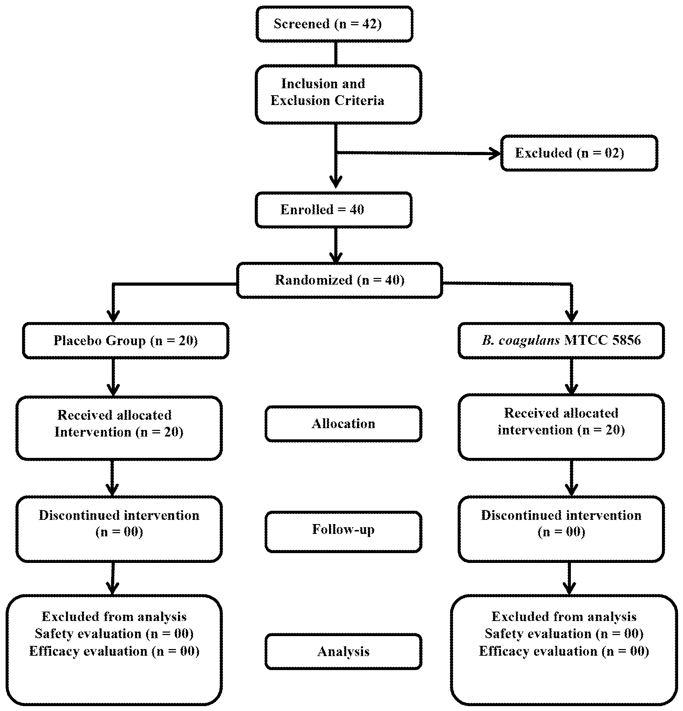

**Fig.1** shows the flowchart describing the procedures involved in the study.

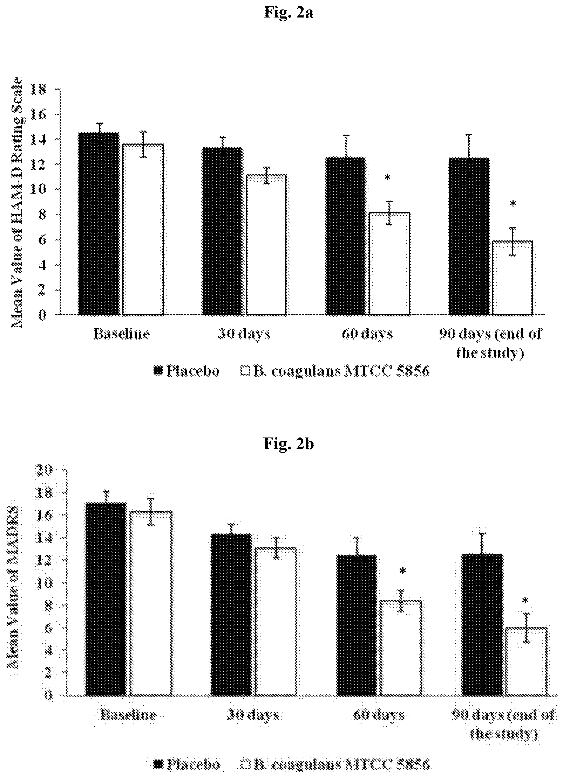

**Fig. 2a** shows the Hamilton Depression Rating Scale (HAM-D), for treatment (*B. coagulans* MTCC 5856) and placebo groups, on a scale of 0 to 20. All the values are expressed as mean ± S.E. ^{∗} indicate statistical significance (p<0.05) between placebo and treatment groups and also between baseline and 60th & 90th day of study.
**Fig. 2b** shows the Montgomery-Asberg Depression rating for treatment (*B. coagulans* MTCC 5856) and placebo groups on a scale of 0 to 20. All the values are expressed as mean ± S.E. ^{∗} indicate statistical significance (p<0.05) between placebo and treatment groups and also between baseline and 60th & 90th day of study.

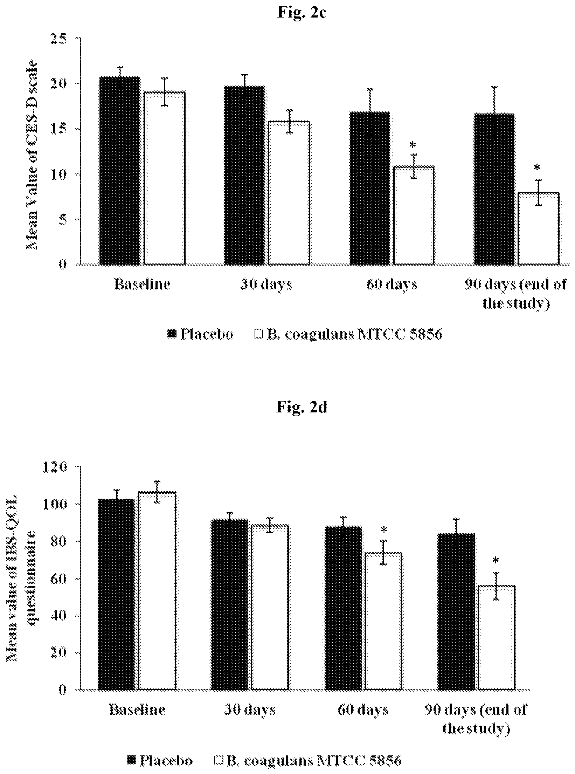

**Fig. 2c** shows the Center for Epidemiologic Studies Depression score for treatment (*B. coagulans* MTCC 5856) and placebo groups, on a scale of 0 to 30. All the values are expressed as mean ± S.E. ^{∗} indicate statistical significance (p<0.05) between placebo and treatment groups and also between baseline and 60th & 90th day of study.
**Fig. 2d** shows the IBS - Quality of Life score for treatment (*B. coagulans* MTCC 5856) and placebo groups on a scale of 150. High QOL value indicates poor quality of life. All the values are expressed as mean ± S.E. ^{∗} indicate statistical significance (p<0.05) between placebo and treatment groups and also between baseline and 60th & 90th day of study.

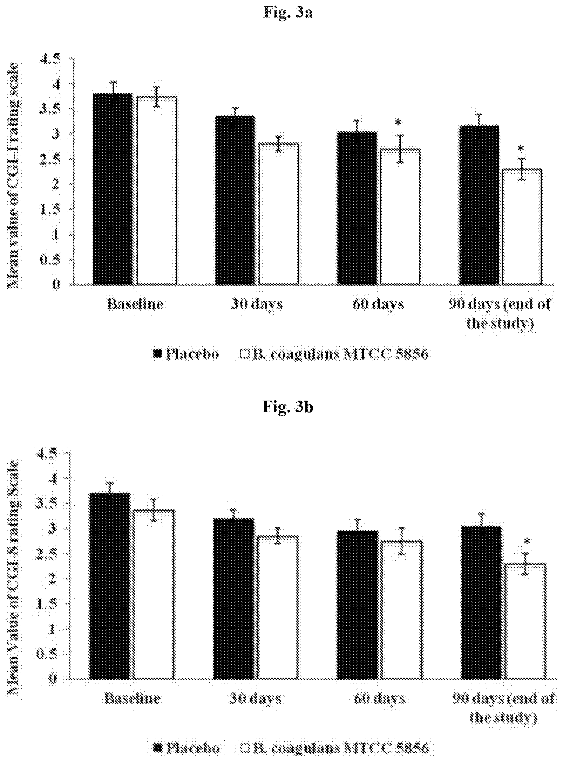

**Fig. 3a** shows the Clinical Global Impression - Improvement Rating for treatment (*B. coagulans* MTCC 5856) and placebo groups on a scale of 0 to 5. All the values are expressed as mean ± S.E. ^{∗} indicate statistical significance (p<0.05) between placebo and treatment groups and also between baseline and 60th & 90th day of study.
**Fig. 3b** shows the Clinical Global Impression - Severity Rating for treatment (*B. coagulans* MTCC 5856) and placebo groups on a scale of 0 to 5. All the values are expressed as mean ± S.E. ^{∗} indicate statistical significance (p<0.05) between placebo and treatment groups and also between baseline and 90th day of study.

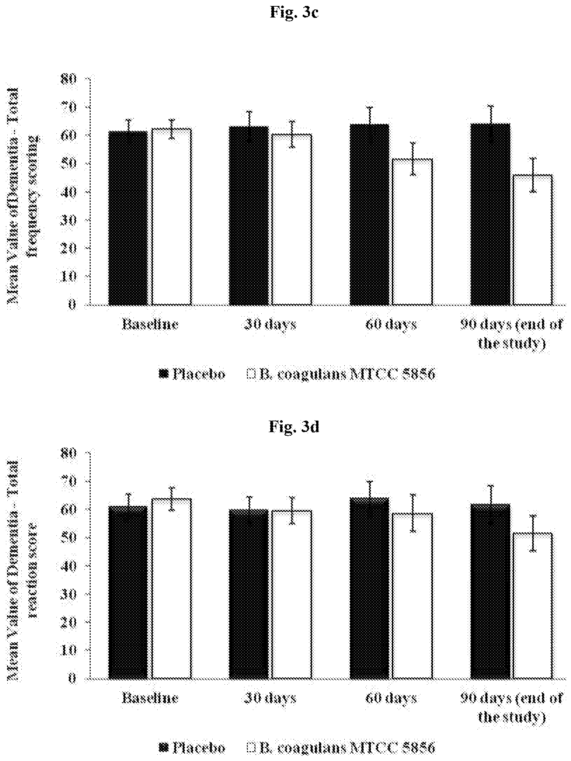

**Fig. 3c** shows the Dementia - Total Frequency Scoring for treatment (*B. coagulans* MTCC 5856) and placebo groups on a scale of 0 to 100. All the values are expressed as mean ± S.E
**Fig. 3d** shows the Dementia Total Reaction Scoring for treatment (*B. coagulans* MTCC 5856) and placebo groups on a scale of 0 to 100. All the values are expressed as mean ± S.E.

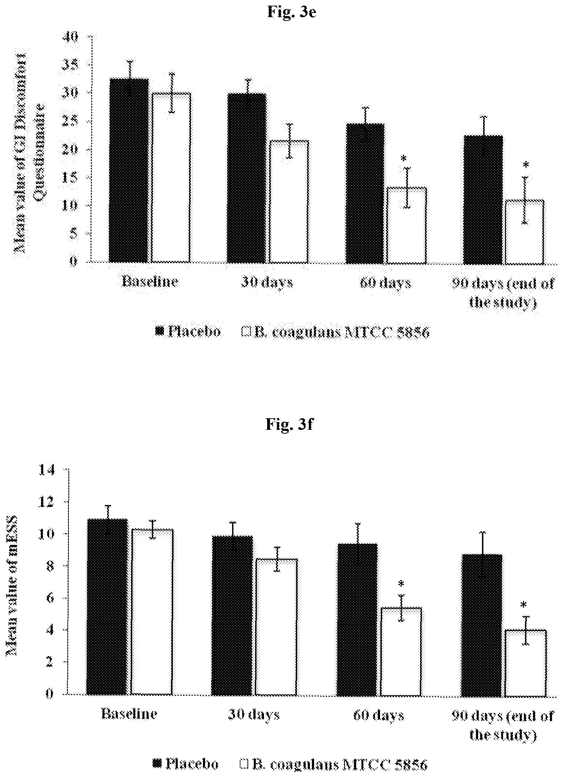

**Fig. 3e** shows the Gastrointestinal Discomfort Questionnaire for treatment (*B. coagulans* MTCC 5856) and placebo groups on a scale of 0 to 40. Low value indicates less GI discomfortness. All the values are expressed as mean ± S.E. ^{∗} indicate statistical significance (p<0.05) between placebo and treatment groups and also between baseline and 60th & 90th day of study.
**Fig. 3f** shows the Modified Epworth Sleepiness Scale for treatment (*B. coagulans* MTCC 5856) and placebo groups on a scale of 0 to 15. All the values are expressed as mean ± S.E. ^{∗} indicate statistical significance (p<0.05) between placebo and treatment groups and also between baseline and 60th & 90th day of study.

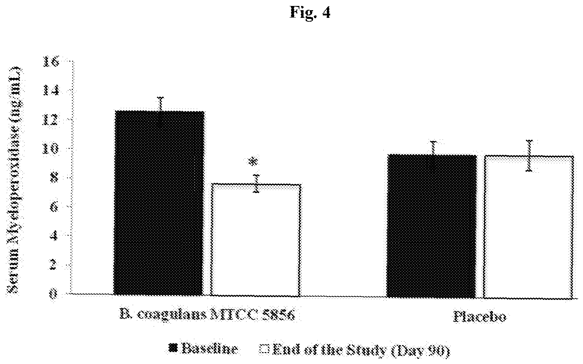

**Fig. 4** shows the effect of *B. coagulans* MTCC 5856 supplementation on the serum myeloperoxidase activity. The figure also describes the serum myeloperoxidase activity at the baseline and end of the study (day 90) of both treatment and placebo groups. the values are expressed as mean ± S.E. ^{∗} indicate statistical significance (p<0.01) between the groups and also between baseline and end of the study (day 90).

### DESCRIPTION OF THE MOST PREFERRED EMBODIMENTS

Described herein is a method of therapeutically managing major depressive disorder in mammals, said method comprising step of administering an effective dose of *Bacillus coagulans* MTCC 5856 to mammals experiencing symptoms of major depressive disorder. Also described is The use of *Bacillus coagulans* MTCC 5856 for therapeutic management of symptoms of major depressive disorder associated with Irritable Bowel Syndrome (IBS). *Bacillus coagulans* MTCC 5856 is preferably administered at a dose of 2 × 10⁹ cfu (spores)/day. Preferably, the mammal is human. The symptoms of major depressive disorder are one selected from the group consisting of sleep disorder and dementia.

Also described is a method of reducing serum myeloperoxidase levels in mammals experiencing symptoms of major depressive disorder said method comprising step of administering an effective dose of *Bacillus coagulans* MTCC 5856 to mammals experiencing symptoms of major depressive disorder. Also described is use of *Bacillus coagulans* MTCC 5856 for reducing serum myeloperoxidase levels in mammals experiencing symptoms of major depressive disorder associated with Irritable Bowel Syndrome (IBS). In particular, *Bacillus coagulans* MTCC 5856 may be administered at a dose of 2 × 10⁹ cfu (spores)/day. In a preferred embodiment, the mammal is human.

The specific examples included herein below illustrate the most preferred embodiments of the present invention.

### [Para0022] Example 1

### [Para0023] Materials and Methods

### [Para0024] Product description

*B. coagulans* MTCC 5856 tablets (600 mg) contained 2 billion spores (333.33 mg), microcrystalline cellulose, starch, sodium starch glycolate and magnesium stearate. Placebo tablets contained dibasic calcium phosphate, microcrystalline cellulose, starch, and magnesium stearate, No differences in color, taste, texture or packaging were detectable between the two products. Tablets were sealed in identically-appearing, high-density polyethylene bottles with desiccant. Viable spore count of *B. coagulans MTCC* 5856 was determined by following pour plate method as per described previously (Majeed et al, Nutr J. 2016, 15:21). Analysis was performed twice in triplicate. Average means of spore viable counts was expressed in cfu/g.

### [Para0026] Ethics and informed consent

This trial was conducted in accordance with the clinical research guidelines established by the Drugs and Cosmetics Act, 1940 of India, Drugs and Cosmetics Rules, 1945 of India, Ethical Guidelines for Biomedical Research on Human Participants, 2006 of Indian Council of Medical Research (ICMR) in India, the principles enunciated in the Declaration of Helsinki (Edinburgh, 2000) and the ICH - harmonized tripartite guideline regarding Good Clinical Practice (GCP). Written and oral information about the study in a language understandable by the subject was provided to all subjects. This study was registered at Clinical Trials Registry- India (www.ctri.nic.in) under the identifier CTRI/2015/05/005754 on 06 May 2015. There were no changes to the methods or planned endpoints after study initiation.

### [Para0028] Participants

Subjects were included in the study if indicated "Yes" to all of the inclusion criteria and "No" to all of the exclusion criteria.

**Inclusion Criteria.** (1) Male and/or female subjects ranging in age between 20 to 65 years. (2) Fulfilling Rome III Diagnostic Criteria (Drossman, Gastroenterology. 2006, 130:1377-1390) for Functional IBS (Functional Diarrhea). Criterion fulfilled for the last 3 months with symptom onset at least 6 months prior to diagnosis. (a) Recurrent abdominal pain or discomfort (uncomfortable sensation not described as pain) at least 3 days/month in the last 3 months associated with two or more of the following: (i). Improvement with defecation; (ii). Onset associated with a change in frequency of stool; (iii). Onset associated with a change in form (appearance) of stool; (b) Recurrent feeling of bloating or visible distension at least 3 days/month in the last 3 months; (c) Loose (mushy) or watery stools without pain occurring in at least 75% of stools. (3) Willingness to follow the protocol requirement as evidenced by written, informed consent. (4) Newly diagnosed patients with mild to moderate IBS in severity with possible sleep, pain and dementia associated comorbidities. (5) Willingness to complete subject diaries and study questionnaires. (6) Agree not to use any medication (prescription and over the counter), including vitamins and minerals, during the course of this study. (7) Agree not to use any yogurt during the course of this study. (8) Subjects whose blood chemistries are within a normal range or not considered clinically significant if outside the normal range. (9) Subject's assurance that they have not taken antibiotics or other Supplements whose primary site of action is in the GIT for a period up to 1 month prior to the start of the study. (10) Willing to come for regular follow-up visits.

**Exclusion Criteria.** (1) Any clinically significant medical history, medical finding or an ongoing medical or psychiatric condition exists which in the opinion of the investigator could jeopardize the safety of the subject, impact validity of the study results or interfere with the completion of study according to the protocol. (2) Significant abnormal findings as determined by baseline history, physical examination, vital signs (blood pressure, pulse rate, respiration rate) hematology, serum chemistry, urinalysis. (3) History or presence of significant alcoholism or supplement/drug abuse in the past one year. (4) Any medical or surgical conditions, which might significantly, interfere with the gastro intestinal tract, liver, kidneys, and/or blood-forming organs. (5) History of cardiovascular, renal, hepatic, asthma, glaucoma, pulmonary, neurologic, metabolic or psychiatric disease. (6) Participation in a clinical study during the preceding 90 days. (7) History of malignancy or other serious disease. (8) Any contraindication to blood sampling. (9) Smoking or Consumption of tobacco products. (10) Blood or blood products donated in past 30 days prior to study supplement administration. (11) Pregnant female subjects and lactating women. (12) Prior surgical therapy for obesity. (13) Patients using yogurt in their daily meal.

### [Para0032] Trial Design

The disposition of the study participant shown in **Fig. 1**. This randomized, multicenter, double-blind, placebo controlled, parallel-group, clinical trial was conducted in 40 outpatient clinics and hospitals in India between April 2015 and October 2015. This clinical trial was conducted at three different sites [(i) Life Care Hospital, Bangalore, India. (ii) Sri Venkateshwara Hospital, Bangalore, India. (iii) Sapthagiri Institute of Medical Sciences and Research Center, Bangalore, India). The sample size of the study was 40, with 20 subjects randomized to each of the two study arms in a double-blinded manner at a 1:1 ratio. The subjects were blinded and received dosing as per randomization code provided at each site by an authorized person independently. Compliance with study supplement was reviewed at each visit. This was by examination of the returned supplement. All accountability records were incorporated into the investigator's study file. History of any medications being used currently were elicited and documented. The subjects were followed up regularly for all concomitant dosing from the time of screening till the follow up visit was captured and recorded. The patients were instructed against the use of any kind of yoghurt during the study duration. The daily food intake of the patients was recorded in the patient diaries provided to them at Visit 1 (Day 0). The same was checked and verified at subsequent visits by the investigators. The study consisted of a 90 day intervention period. Subjects met with the investigational team for screening, the baseline/randomization visit, day 30, day 60, day 90 and day 105. A description of Visits 1, 2, 3 and 4 with schedule of events are provided in **Table 1**.

**Table 1 Schedule of events**

| **Procedure** | **Screening** | **Visit 1 (Day 0) Baseline** | **Visit 2 (Day 30)** | **Visit 3 (Day 60)** | **Visit 4 (Day 90) Final visit** | **Follow Up Visit (At least 15 days from the last visit)** |
|---|---|---|---|---|---|---|
| Informed consent | X | | | | | |
| Medical history | X | | | | | |
| Physical examination | X | X | X | X | X | |
| Demographics^{a} | X | X | X | X | X | |
| Vital Signs | X | X | X | X | X | |
| Hematology | X | | | | X | |
| Serum Chemistry | X | | | | X | |
| Myeloperoxidase ^{b} | | X | | | X | |
| Urine pregnancy test ^{c} | X | | | | | |
| Randomization | | X | | | | |
| IP Dispensing and Dosing | | X | X | X | | |
| Gastrointestinal Discomfort Questionnaire | | X | X | X | X | |
| Irritable Bowel SyndromeQuality of Life Questionnaire | | X | X | X | X | |
| HAM -D scale | | X | X | X | X | |
| MADRS | | X | X | X | X | |
| CGI-I & CGI-S | | X | X | X | X | |
| CES-D | | X | X | X | X | |
| Dementia checklist | | X | X | X | X | |
| Return of Unused IP | | | X | X | X | |
| Adverse events | | X | X | X | X | X |
| Concomitant medications | X | X | X | X | X | X |

| | | | | | | |
|---|---|---|---|---|---|---|
| ^{a} Age at screening only. ^{b} Only for randomized subjects ^{c} Urine pregnancy test at screening and on early termination, if any. | | | | | | |

The assigned *B. coagulans* MTCC 5856 (LactoSpore^{®}) study product was double blinded, i.e., neither the subjects nor the study staff knew the treatment group assigned until study completion. Double blinding was accomplished by independent blinding of the dosing bottles.

### [Para0036] Intervention

Newly diagnosed patients who were not on any other treatment for major depression with IBS in the past 3 months were enrolled into the study. All enrolled subjects were asked to self administer one tablet per day (either *Bacillus coagulans* MTCC 5856 or Placebo) at least 30 minutes before a meal, in the morning as a dietary supplement for a period of 90 days.

### [Para0038] Efficacy Outcomes

The primary outcome for this study was the mean 90 day change in depression and IBS symptoms as assessed by the Hamilton Rating Scale for Depression (HAM-D) (Friedrich et al, Clin Ther. 2010, 32: 1221-1233), Montgomery-Asberg Depression rating Scale (MADRS) Bravo et al, Proc Natl Acad Sci USA, 2011, 108: 16050-16055; Kim et al, Aliment Pharmacol Ther. 2003, 17:895-904), sleep quality and depressive symptom severity using 11-item Center for Epidemiological Studies-Depression Scale (CES-D) (Kim et al, Neurogastroenterol Motil. 2005, 17: 687-696) and Irritable Bowel Syndromequality of life questionnaire (IBS-QOL) (Tsuchiya et al, Chin J Dig Dis. 2004, 5:169-174). Additionally, secondary efficacy assessments included change in Clinical Global Impression- Improvement rating Scale (CGI-I) (Baron, Postgrad Med. 2009, 121: 114-118), Clinical Global Impression Severity rating Scale (CGI-S) (Baron, Postgrad Med. 2009, 121: 114-118), Dementia - Revised Memory and Behaviour Problem Checklist (RMBPC) (Jensen et al, BMC Immunol. 2010,11:15*),* Gastrointestinal Discomfort Questionnaire (GI-DQ) (O'Mahony et al, Gastroenterology. 2005, 128: 541-551) and Modified Epworth Sleepiness Scale (mESS) (Johns, Sleep. 1991, 14: 540-545) from the baseline till the end of study as subjective test.

### Bioassays

Along with subjective assessments (questionnaires), serum Myeloperoxidase, an inflammatory biomarker was also analysed for both the groups (*B. coagulans* MTCC 5856 and placebo) at the baseline and end of the study. Serum levels of myeloperoxidase in the blood samples were measured by the implementation of enzyme-linked immunosorbent assay (ELISA) techniques according to manufacturer directions. (Myeloperoxidase (human) EIA Kit, Code, 585001, Cayman Chemicals Company, MI, USA)

### Safety Outcomes

Safety of the study was assessed considering the occurrence of adverse events, safety blood parameters, and the follow-up of vital signs (blood pressure and heart rate). Laboratory data was summarized by presenting summary statistics of raw data and change from baseline values to end of study in laboratory values relative to normal reference limits. Descriptive physical examination such as abdomen, extremities, general appearance, head, ear, nose, throat, heart, lungs and neurological were monitored at the screening, day 0, day 30, day 60, day 90 and day 120 for the safety evaluation. Spontaneously reported or observed AEs were assessed at all post-screening study visits. AEs were evaluated in terms of intensity (mild, moderate, or severe) and possible relationship to the study product.

### Data reporting and management

All data was reported in the respective hospital records that were then transcribed onto the Case Report Forms (CRFs). All data reported was first reviewed by the respective investigators present at the three sites and then entered by the clinical research coordinators onto the CRFs. This data entered in the CRFs was again verified by the investigators a second time. It was ensured that the source data matches with the data entered in the CRFs complying with the GCP guidelines on source data verification. Data collection during this clinical study and preparation of the data for analysis was conducted by separate and independent functional groups. Standard procedures ensured all CRFs are tracked and properly routed. The training of all the end users and clinical data management associates pertaining to the database entry and validation process was documented. The data entry operator transcribed the data from the paper CRF to the database. Data validation was conducted by the data manager. The database was locked post reconciliation of all data. The locked database was provided to the statistician who was independent of the study team. The data was then analyzed statistically.

### Statistical Analysis

Statistical Analysis Software (SAS) of version 9.2 software was used for data analysis here. Paired '*t*' test, Analysis of Covariance (ANCOVA) and Wilcoxon signed rank sum test were used for appropriate data set variables to reach the best possible statistical conclusion between the *B. coagulans* MTCC 5856 receiving and Placebo receiving groups. The baseline descriptors were summarized as means and standard deviations for continuous variables and as frequencies and percentages for categorical variables. Last Observation Carry Forward (LOCF) method was followed for efficacy evaluations of subjects, whose data was not available in the last/final visit. No formal sample size calculation was performed.

### Results

### Patient disposition and characteristics

A total of 42 subjects were screened and 40 were enrolled into the study. There were no patient withdrawals or dropouts in this study. Out of 42 subjects screened, 40 were enrolled into the study and randomized into *B. coagulans* MTCC 5856 and placebo groups in 1:1 ratio. Treatment compliance across various visits and overall treatment compliance for the whole study indicates 24 (60 %) patients met 100 % treatment compliance on visit 4 (day 90) and on an average 66.7 % met with 100 % treatment compliance during the whole study period. At baseline visit (Day 0), no significant difference was observed between the two treatment groups in subject demographics (**Table 2**). On the day of screening, the mean age of all the enrolled subjects was 35.8 ± 10.91, mean weight was 64.9 ± 11.20 Kg and mean height was 158.8 ± 8.24 cm. The mean BMI was 25.6 ± 4.42 Kg/m² with 6 males (15%) and 34 females (85%) enrolled into the study. While one subject was non user of tobacco or tobacco products along alcoholic drinking history, 37 subjects (92.50%) were neither tobacco users nor alcoholic drinkers. Remaining subjects were occasional smokers and alcoholic drinkers. None of the enrolled subjects had abnormal medical history, except for gastro-intestinal. Around 03 subjects (7.50%) had earlier GI related medical history which had no interference with IBS. There was a statistically significant change (p<0.058) in the body weight in the group that received *B. coagulans*MTCC 5856 over placebo. However, no significant change in their respective BMI values was observed in both the groups.

**Table 2 Demographics and Baseline Clinical Characteristics**

| | | **Placebo (n=20)** | | ***B. coagulans* MTCC 5856 (n=20)** | |
|---|---|---|---|---|---|
| Sex, n (%) | | | | | |
| | Female | 17 | (85) | 17 | (85) |
| | Male | 03 | (15) | 03 | (15) |
| Age (years), mean (SD) | | 43.88 ± 9.85 | 40.36 ± 10.28 | | |
| Height (cm), Mean(SD) | | | | | |
| Body Mass Index (kg/m2) | | 157.39 ± 8.49 | 160.1 ± 7.87 | | |
| | | 25.9 ± 4.49 | | 25.4 ± 4.46 | |
| Smokers, n (%) | | | | | |
| | Ex-Smoker | 18 | (90) | 19 | (95) |
| | Non-Smoker | 01 | (5) | 00 | |
| | Smoker | 01 | (5) | 01 | (5) |
| Race, n (%) | | | | | |
| | Central American | 00 | | 00 | |
| | East Asian | 00 | | 00 | |
| | South Asian | 20 | (100) | 20 | (100) |
| | South American | 00 | | 00 | |
| | South East Asian | 00 | | 00 | |
| | Western European | 00 | | 00 | |
| | White | 00 | | 00 | |
| Alcohol use | | | | | |
| | Non Drinker | 01 | (5) | 00 | |
| | Past Drinker | 18 | (90) | 19 | (95) |
| | Occasional Drinker | 01 | (5) | 01 | (5) |
| | Current Drinker | 00 | | 00 | |
| Baseline data, mean (SD) | | | | | |
| | IBS-QOL | 102.6 ± 21.11 | | 106.4 ± 23.44 | |
| | CGI-I | 3.8 ± 1.01 | | 3.7 ± 0.87 | |
| | CGI-S | 3.7 ± 0.92 | | 3.4 ± 0.96 | |
| | HAM -D | 14.5 ± 3.41 | | 13.6 ± 4.41 | |
| | MADRS | 17.1 ± 4.63 | | 16.3 ± 5.40 | |
| | CES-D | 20.7 ± 4.86 | | 19.1 ± 5.25 | |
| | Dementia - Total frequency scoring | 61.3 ± 19.11 | | 62.3 ± 17.08 | |
| | Dementia - Total reaction scoring | 61.0 ± 19.83 | | 63.8 ± 17.57 | |
| | mESS | 10.9 ± 2.99 | | 10.3 ± 2.43 | |
| | GI-DQ | 32.5 ± 13.88 | | 30.1 ± 15.07 | |

| | | | | | |
|---|---|---|---|---|---|
| CES-D, Center for Epidemiological Studies Depression Scale; CGI-I, Clinical Global Impression- Improvement rating Scale; CGI-S, Clinical Global Impression Severity rating Scale; CI, confidence interval GI-DQ, Gastrointestinal Discomfort Questionnaire; HAM-D, Hamilton Rating Scale for Depression; IBS-QOL, Irritable Bowel Syndromequality of life questionnaire; MADRS, Montgomery-Asberg Depression Rating Scale; mESS, Modified Epworth Sleepiness Scale; Values expressed as mean ± S.D | | | | | |

### Efficacy Evaluation

As IBS symptoms are closely associated with clinical symptoms of depression, change in depression symptoms and IBS-quality of life were analyzed throughout the study period as primary efficacy measures. The '*p*' value suggests that there was a statistically significant change in these symptoms from baseline to final visit, between the placebo and *B. coagulans*MTCC 5856 arms. At baseline visit (Day 0), no significant difference was observed between the two treatment groups in clinical characteristics (efficacy parameters) (**Table 2**). Statistical analysis using Analysis of CoVariance (ANCOVA) showed the primary efficacy parameters were found to be statistically significant (p <0.01) between the *B. coagulans* MTCC 5856 and placebo groups (**Table 3**). This was largely the result of benefit in improving the symptoms of depression and IBS quality of life. The significant change in the score of HAM-D, MADRS, CES-D and IBS-QOL is the most relevant parameters to evaluate the clinical significance of depression and IBS (Friedrich et al, Clin Ther. 2010, 32: 1221-1233; Bravo et al, Proc Natl Acad Sci USA, 2011, 108: 16050-16055; Kim et al, Aliment Pharmacol Ther. 2003, 17:895-904; Kim et al, Neurogastroenterol Motil. 2005, 17: 687-696; Tsuchiya et al, Chin J Dig Dis. 2004, 5:169-174). Similarly, all secondary efficacy parameters were also found to be statistically significant between the *B. coagulans* MTCC 5856 and placebo groups except for "Dementia total reaction scoring" (**Table 3**).

**Table 3 Summary of efficacy outcomes at the end of study (day 90): (full analysis set, last observation carried forward, ANCOVA model, 95% CI).**

| **Efficacy Parameters** | **Placebo (n = 20)** | ***B. coagulans* MTCC 5856 (n = 20)** | ***p*-value** |
|---|---|---|---|
| **Primary efficacy outcomes** | | | |
| HAM -D | 12.5 ± 8.70 | 5.9 ± 4.88 | 0.007^{∗} |
| MADRS | 12.6 ± 8.00 | 6.0 ± 5.79 | 0.007^{∗} |
| CES-D | 16.7 ± 13.03 | 8.0 ± 6.17 | 0.013^{∗} |
| IBS-QOL | 84.1 ± 34.67 | 56.1 ± 31.26 | 0.0109^{∗} |

| **Secondary efficacy outcomes** | | | |
|---|---|---|---|
| CGI-I | 3.2 ± 1.09 | 2.3 ± 0.92 | 0.01^{∗} |
| CGI-S | 3.1 ± 1.05 | 2.3 ± 0.92 | 0.022^{∗} |
| Dementia - Total frequency scoring | 64.0 ± 28.26 | 45.9 ± 26.42 | 0.012^{∗} |
| Dementia - Total reaction scoring | 61.8 ± 29.94 | 51.6 ± 28.19 | 0.118 |
| GI-DQ | 22.9 ± 14.55 | 11.4 ± 18.23 | 0.045^{∗} |
| mESS | 8.9 ± 6.24 | 4.2 ± 3.92 | 0.01^{∗} |

| | | | |
|---|---|---|---|
| ANCOVA, analysis of covariance; CES-D, Center for Epidemiological Studies Depression Scale; CGI-I, Clinical Global Impression- Improvement rating Scale; CGI-S, Clinical Global Impression Severity rating Scale; CI, confidence interval GI-DQ, Gastrointestinal Discomfort Questionnaire; HAM-D, Hamilton Rating Scale for Depression; IBS-QOL, Irritable Bowel Syndromequality of life questionnaire; MADRS, Montgomery-Asberg Depression Rating Scale; mESS, Modified Epworth Sleepiness Scale; ^{∗}*p value significant* (*<0.05*) Values expressed as mean ± S.D | | | |

Furthermore, comparative mean values of efficacy assessments between *B. coagulans* MTCC 5856 and placebo groups across various visits (baseline, day 30, day 60 and day 90) are presented for primary efficacy parameters (**Figs. 2a, b****,** **c, d**) and secondary efficacy parameters (**Figs. 3a, b****,** **c, d****,** **e, f**). Patients who were on *B. coagulans* MTCC 5856 had statistically significant difference for the efficacy parameters on day 60 and maintained till end of the study when compared with placebo (**Fig. 2** **and** **Fig. 3**). *B. coagulans* MTCC 5856 was also found to be very beneficial for sleeplessness and to a less extent dementia in the current set of patients (**Table 3 and** **Figs. 3c**,**d****,****f**).

### Bioassay

The level of serum myeloperoxidase was significantly reduced from the baseline to the end of study (day 90) in patients receiving *B. coagulans* MTCC 5856 2 × 10⁹ spores (cfu)/day (p<0.01) (**Fig. 4**). However, no significant change of the level of serum myelo-peroxidase was observed in the placebo group (p>0.05) reduced from the baseline to the end of study (day 90) **(****Fig.4****).**

### Safety Evaluations

Vital signs such as Blood Pressure, Respiratory Rate, Pulse Rate and any abnormal lab/diagnostic parameters were considered for safety evaluations. No clinically significant changes were recorded for descriptive physical examination in both the group (*B. coagulans* MTCC 5856 and placebo group). Further, no clinically significant abnormal lab values (biochemistry and haematology) were identified and no statistically significant changes in the vitals were observed from the baseline to final visit (**Table 4 and Table 5**). There were no Serious Adverse Events or Significant Adverse Events noticed in this study. There was one adverse event reported with fever and weakness. The site investigator classified this adverse event as mild with no plausible relationship with the product and the adverse event reported to have been resolved without the use of any concomitant medication(s). The unblinding of study product code towards end of the study revealed that the patient with the adverse event belonged to the placebo group.

**Table 4 Biochemistry and haematology values between two treatment groups**

| **Lab Parameter (Units)** | **Visit** | **Placebo (n=20)** | ***B. coagulans* MTCC 5856 (n=20)** | **Normal range** |
|---|---|---|---|---|
| Alanine aminotransferase (IU/L) | Screening | ± 8.19 | | |
| | Final Visit | 26.9 ± 26.87 | 31.0 ± 25.18 | |
| Albumin (g/dL) | Screening | 4.5 ± 0.33 | 4.6 ± 0.32 | 3.5 to 5.2 |
| | Final Visit | 4.4 ± 0.32 | 4.3 ± 1.01 | |
| Alkaline Phosphatase (U/L) | Screening | 206.2 ± 84.44 | 210.1 ± 97.16 | 53 to 128 |
| | Final Visit | 196.1 ± 72.90 | 214.3 ± 75.42 | |
| Aspartate aminotransferase (IU/L) | Screening | 25.1 ± 5.95 | 26.4 ± 7.00) | 0 to 40 |
| | Final Visit | 27.1 ± 16.69 | 27.3 ± 13.79 | |
| Blood urea nitrogen (mg/dL) | Screening | 12.9 ± 3.00 | 13.0 ± 5.87 | 5.0 to 24 |
| | Final Visit | 11.5 ± 2.75 | 11.4 ± 2.38 | |
| Fasting Blood Sugar (mg/dL) | Screening | 104.5 ± 51.03 | 114.1 ± 69.14 | 70 to 110 |
| | Final Visit | 105.9 ± 56.70 | 117.2 ± 78.78 | |
| LDL Cholesterol (mg/dL) | Screening | 121.6 ± 24.67 | 127.2 ± 42.56 | Up to 140 |
| | Final Visit | 123.0 ± 31.43 | 121.1 ± 44.51 | |
| Potassium (mEq/L) | Screening | 4.8 ± 0.41 | 4.7 ± 0.45 | 3.5 to 5.2 |
| | Final Visit | 5.7 ± 1.76 | 7.3 ± 7.44 | |
| Serum Creatinine (mg %) | Screening | 0.8 ± 0.12 | 1.2 ± 1.84 | 0.6 to 1.4 |
| | Final Visit | 1.2 ± 2.07 | 0.8 ± 0.12 | |
| Sodium (mEq/L) | Screening | 140.8 ± 2.63 | 133.2 ± 30.13 | 136 to 145 |
| | Final Visit | 138.5 ± 2.89 | 132.6 ± 21.55 | |
| Total Bilirubin (mg/dL) | Screening | 1.1 ± 0.44 | 1.4 ± 0.72 | 0.1 to 1.2 |
| | Final Visit | 1.3 ± 0.57 | 1.6 ± 1.14 | |
| Total Protein (g/dL) | Screening | 7.6 ± 0.53 | 7.6 ± 0.44 | 6.22 to 8.0 |
| | Final Visit | 7.4 ± 0.46 | 7.4 ± 0.56 | |
| Erythrocyte Count (×10⁶ cells) | Screening | 4.4 ± 0.61 | 4.6 ± 0.53 | 4.0 to 6.5 |
| | Final Visit | 4.6 ± 0.65 | 4.6 ± 0.39 | |
| Haematocrit (%) | Screening | 37.9 ± 5.37 | 41.5 ± 3.60 | 40 to 50 |
| | Final Visit | 37.0 ± 5.63 | 38.1 ± 8.72 | |
| Haemoglobin (gm %) | Screening | 11.9 ± 2.00 | 13.2 ± 1.52 | 11 to 16 |
| | Final Visit | 11.7 ± 2.06 | 12.5 ± 2.15 | |
| Luekocyte Count (Cells cu. mm-¹) | Screening | 8562.5 ± 1930.07 | 9233.3 ± 2369.35 | 4000 to 11, 000 |
| | Final Visit | 8575.0 ± 2879.49 | 8157.5 ± 2413.95 | |
| Platelet Count (×10⁵ per cu. mm) | Screening | 2.7 ± 0.97 | 2.8 ± 0.80 | 1.5 to 4.5 |
| | Final Visit | 2.4 ± 0.91 | 2.3 ± 0.70 | |

| | | | | |
|---|---|---|---|---|
| Values expressed as mean ± S.D | | | | |

**Table 5 Change in mean Vital signs from baseline to the end of study (90 days)**

| Vital Parameter | Supplements | Baseline | Day 90 (end of the study) | Change | p-value |
|---|---|---|---|---|---|
| Systolic Blood Pressure (mmHg) | *B. coagulans* MTCC 5856 | 122.5 | 121.0 | -1.50 | 0.2674 |
| | Placebo | 123.5 | 120.0 | -3.50 | 0.0308 |
| Diastolic Blood Pressure (mmHg) | *B. coagulans* MTCC 5856 | 77.0 | 79.5 | 2.50 | 0.1713 |
| | Placebo | 80.5 | 80.0 | -0.50 | 0.7157 |
| Pulse Rate (Beats per minute) | *B. coagulans*MTCC 5856 | 74.6 | 74.6 | 0.00 | 1.0000 |
| | Placebo | 76.1 | 74.5 | -1.55 | 0.0841 |
| Heart Rate (Beats per minute) | *B. coagulans*MTCC 5856 | 74.7 | 74.8 | 0.10 | 0.8660 |
| | Placebo | 75.8 | 74.8 | -1.00 | 0.1467 |
| Respiratory Rate (Breaths per minute) | *B. coagulans*MTCC 5856 | 21.1 | 21.1 | 0.00 | 1.0000 |
| | Placebo | 21.4 | 20.8 | -0.60 | 0.1240 |
| Weight (kg) | *B. coagulans*MTCC 5856 | 65.7 | 66.7 | 1.04 | 0.0589^{∗} |
| | Placebo | 63.1 | 64.0 | 0.91 | 0.0785 |
| Body Mass Index (kg/m²) | *B. coagulans*MTCC 5856 | 25.6 | 25.7 | 0.01 | 0.8956 |
| | Placebo | 25.7 | 25.8 | 0.01 | 0.8969 |

| | | | | | |
|---|---|---|---|---|---|
| ^{∗} *p-value is estimated from Paired t-test, p<0.05 considered as significant* | | | | | |

### Conclusion

The clinical study concluded that *Bacillus coagulans* MTCC 5856 at a dose of 2 × 10⁹ spores (cfu) per day demonstrated efficacy for the treatment of patients experiencing symptoms of major depressive disorder associated with IBS. Furthermore, supplementation of *Bacillus coagulans* MTCC 5856 improved the depression and IBS symptoms which were also clinically meaningful. The data of the study concluded that *Bacillus coagulans* MTCC 5856 may be an alternative approach for the management of major depressive disorder in IBS patients.

## Claims

1. *Bacillus coagulans* MTCC 5856 for use in a method of therapeutically managing major depressive disorder associated with Irritable Bowel Syndrome in a mammal, said method consists of step of administering an effective dose of *Bacillus coagulans* MTCC 5856 to said mammal experiencing a symptom of major depressive disorder, wherein the symptom of major depressive disorder is one selected from the group consisting of sleep disorder and dementia.

2. *Bacillus coagulans* MTCC 5856 for use according to claim 1, wherein *Bacillus coagulans* MTCC 5856 is administered at a dose of 2 × 10⁹ cfu (spores)/day.

3. *Bacillus coagulans* MTCC 5856 for use according to claim 1, wherein the mammal is a human.

4. *Bacillus coagulans* MTCC 5856 for use according to claim 1, wherein said symptom of major depressive disorder associated with Irritable Bowel Syndrome in said mammal is therapeutically managed by reducing serum myeloperoxidase level in said mammal by the administering of the effective dose of *Bacillus coagulans* MTCC 5856 to said mammal.

5. *Bacillus coagulans* MTCC 5856 for use according to claim 4, wherein *Bacillus coagulans* MTCC 5856 is administered at a dose of 2 × 10⁹ cfu (spores)/day.

6. *Bacillus coagulans* MTCC 5856 for use according to claim 4, wherein the mammal is a human.

7. *Bacillus coagulans* MTCC 5856 for use in a method of therapeutically managing Irritable Bowel Syndrome associated with major depressive disorder in a mammal, said method consists of step of administering an effective dose of *Bacillus coagulans* MTCC 5856 to said mammal.

8. *Bacillus coagulans* MTCC 5856 for use according to claim 7, wherein a symptom of major depressive disorder is one selected from the group consisting of sleep disorder and dementia.

9. *Bacillus coagulans* MTCC 5856 for use according to claim 7, wherein *Bacillus coagulans* MTCC 5856 is administered at a dose of 2 × 10⁹ cfu (spores)/day.

## Patentansprüche

1. *Bacillus coagulans* MTCC 5856 zur Verwendung in einem Verfahren zur therapeutischen Behandlung einer mit einem Reizdarmsyndrom verbundenen depressiven Störung bei einem Säuger, wobei das Verfahren aus einem Schritt der Verabreichung einer wirksamen Dosis von *Bacillus coagulans* MTCC 5856 an den Säuger besteht, der ein Symptom der depressiven Störung erfährt, wobei das Symptom der depressiven Störung eines ist, das aus der Gruppe ausgewählt ist, die aus Schlafstörung und Demenz besteht.

2. *Bacillus coagulans* MTCC 5856 zur Verwendung nach Anspruch 1, wobei *Bacillus coagulans* MTCC 5856 in einer Dosis von 2 × 10⁹ cfu (Sporen)/Tag verabreicht wird.

3. *Bacillus coagulans* MTCC 5856 zur Verwendung nach Anspruch 1, wobei der Säuger ein Mensch ist.

4. *Bacillus coagulans* MTCC 5856 zur Verwendung nach Anspruch 1, wobei das Symptom der depressiven Störung, die mit dem Reizdarmsyndrom in dem Säuger verbunden ist, therapeutisch behandelt wird, indem der Serum-Myeloperoxidase-Spiegel in dem Säuger durch die Verabreichung der wirksamen Dosis von *Bacillus coagulans* MTCC 5856 an den Säuger verringert wird.

5. *Bacillus coagulans* MTCC 5856 zur Verwendung nach Anspruch 4, wobei *Bacillus coagulans* MTCC 5856 in einer Dosis von 2 × 10⁹ cfu (Sporen)/Tag verabreicht wird.

6. *Bacillus coagulans* MTCC 5856 zur Verwendung nach Anspruch 4, wobei der Säuger ein Mensch ist.

7. *Bacillus coagulans* MTCC 5856 zur Verwendung in einem Verfahren zur therapeutischen Behandlung eines mit einer depressiven Störung verbundenen Reizdarmsyndroms bei einem Säuger, wobei das Verfahren aus einem Schritt der Verabreichung einer wirksamen Dosis von *Bacillus coagulans* MTCC 5856 an den Säuger besteht.

8. *Bacillus coagulans* MTCC 5856 zur Verwendung nach Anspruch 7, wobei ein Symptom der depressiven Störung eines ist, das aus der Gruppe ausgewählt ist, die aus Schlafstörung und Demenz besteht.

9. *Bacillus coagulans* MTCC 5856 zur Verwendung nach Anspruch 7, wobei *Bacillus coagulans* MTCC 5856 in einer Dosis von 2 × 10⁹ cfu (Sporen)/Tag verabreicht wird.

## Revendications

1. *Bacillus coagulans* MTCC 5856 destiné à être utilisé dans un procédé de gestion thérapeutique d'un trouble dépressif majeur associé à un syndrome du côlon irritable chez un mammifère, ledit procédé étant constitué de l'étape d'administration d'une dose efficace de *Bacillus coagulans* MTCC 5856 audit mammifère manifestant un symptôme de trouble dépressif majeur, dans lequel le symptôme de trouble dépressif majeur est un symptôme sélectionné parmi le groupe constitué de trouble du sommeil et de démence.

2. *Bacillus coagulans* MTCC 5856 destiné à être utilisé selon la revendication 1, dans lequel *Bacillus coagulans* MTCC 5856 est administré à une dose de 2 × 10⁹ ufc (spores)/jour.

3. *Bacillus coagulans* MTCC 5856 destiné à être utilisé selon la revendication 1, dans lequel le mammifère est l'homme.

4. *Bacillus coagulans* MTCC 5856 destiné à être utilisé selon la revendication 1, dans lequel ledit symptôme de trouble dépressif majeur associé au syndrome du côlon irritable chez ledit mammifère est géré thérapeutiquement en réduisant le niveau de myéloperoxydase sérique chez ledit mammifère par l'administration de la dose efficace de *Bacillus coagulans* MTCC 5856 audit mammifère.

5. *Bacillus coagulans* MTCC 5856 destiné à être utilisé selon la revendication 4, dans lequel *Bacillus coagulans* MTCC 5856 est administré à une dose de 2 × 10⁹ ufc (spores)/jour.

6. *Bacillus coagulans* MTCC 5856 destiné à être utilisé selon la revendication 4, dans lequel le mammifère est l'homme.

7. *Bacillus coagulans* MTCC 5856 destiné à être utilisé dans un procédé de gestion thérapeutique d'un syndrome du côlon irritable associé à un trouble dépressif majeur chez un mammifère, ledit procédé étant constitué de l'étape d'administration d'une dose efficace de *Bacillus coagulans* MTCC 5856 audit mammifère.

8. *Bacillus coagulans* MTCC 5856 destiné à être utilisé selon la revendication 7, dans lequel un symptôme de trouble dépressif majeur est un symptôme sélectionné parmi le groupe constitué de trouble du sommeil et de démence.

9. *Bacillus coagulans* MTCC 5856 destiné à être utilisé selon la revendication 7, dans lequel *Bacillus coagulans* MTCC 5856 est administré à une dose de 2 × 10⁹ ufc (spores)/jour.
